# FASCICULE DE BREVET EUROPEEN

(11) **EP 2 603 153 B1**
(45) Date de publication et mention de la délivrance du brevet: **22.02.2017**
(21) Numéro de dépôt: 11730307.3
(22) Date de dépôt: 07.07.2011
(51) Int. Cl.: A61B 17/64, F16B 7/04

(54) **DISPOSITIF DE MAINTIEN DU BASSIN**
BECKENKLEMME
PELVIS CLAMP

(30) Priorité: 09.08.2010 CH 12852010
(43) Date de publication de la demande: 19.06.2013
(73) Titulaire: Chirmat Sàrl, 1870 Monthey (CH)
(72) Inventeur: ARLETTAZ, Yvan, CH-1870 Monthey (CH); BONJOUR, Christian, CH-1268 Begnins (CH); PETER, Robin, CH-1246 Corsier (CH)
(74) Mandataire: P&TS SA (AG, Ltd.)
(86) Numéro de dépôt international: PCT/EP2011/061553
(87) Numéro de publication internationale: WO 2012/019835

(56) Documents cités:
- EP-A1- 1 488 750
- US-A- 5 196 012
- US-A- 5 300 071
- US-A- 6 162 222
- US-A1- 2008 086 122
- US-A1- 2009 216 231
- US-A1- 2009 264 884

## Description

### Domaine technique

La présente invention concerne un dispositif de maintien du bassin.

### Etat de la technique

Les dispositifs de maintien du bassin sont des instruments de stabilisation d'urgence pour les lésions et fractures instables de la ceinture pelvienne. Les fractures instables de la ceinture pelvienne peuvent être associées à une perte de sang importante qui peut provoquer un choc hémorragique terminal. Ces dispositifs permettent une réduction et une stabilisation rapide de ces fractures instables de la ceinture pelvienne. Il s'agit donc de dispositifs de grande valeur.

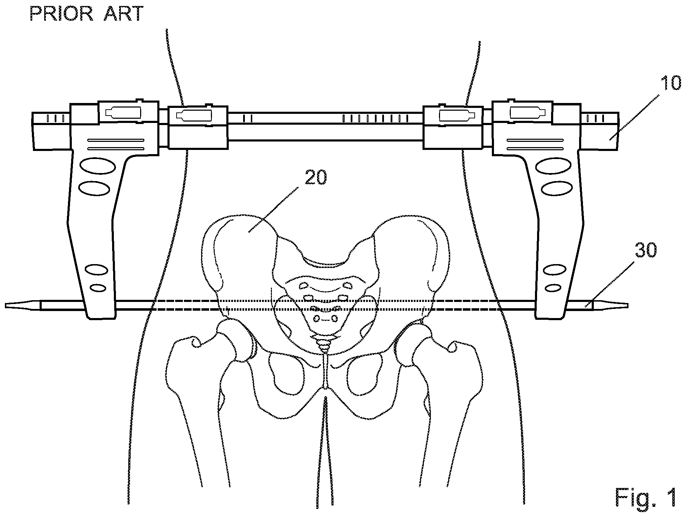

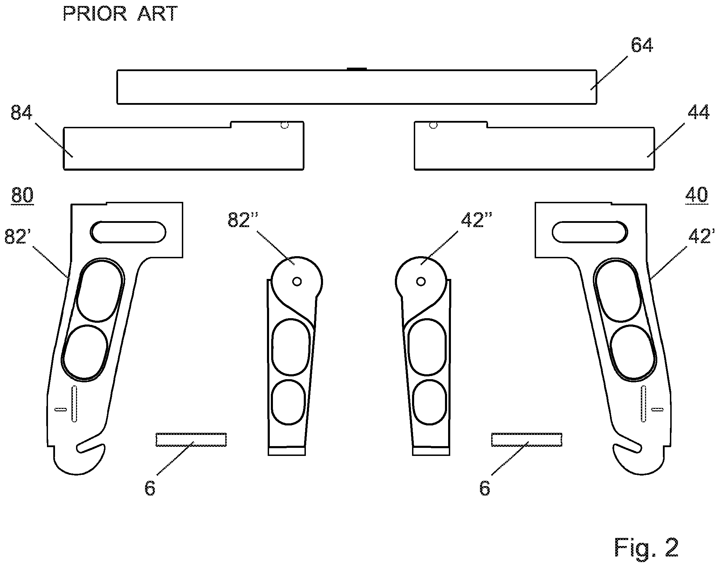

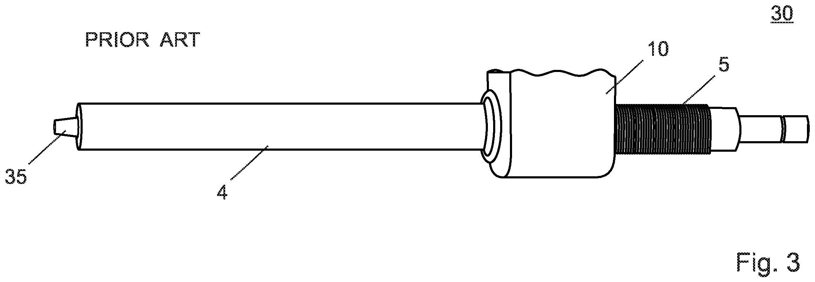

La figure 1 illustre un dispositif 10 de maintien du bassin 20 connu. Comme détaillé sur la figure 2, il comporte trois rails, un rail intérieur 64 et deux rails extérieurs 84, 44 ainsi que des bras de tension 42', 42", 82', 82" avec un mécanisme de verrouillage 30, visible sur la figure 3, composé par une douille filetée et une vis avec le filetage 5. Cette configuration permet un mouvement linéaire des bras de tension sur les rails extérieurs.

Un des inconvénients d'un tel système est lié au nombre élevé de pièces dont il est composé, qui ne permet pas un montage rapide du dispositif, ce qui pourrait être dangereux pour le patient. En outre, après l'usage, le système doit être démonté. Il est nécessaire de bien nettoyer manuellement les rails, les bras de tension et les douilles filetées et, le cas échéant, de remplacer les composants cassés ou déformés. Après le nettoyage il est nécessaire de lubrifier le filetage de la douille, vérifier le bon fonctionnement du dispositif et stériliser toutes les pièces, ce qui requiert du temps et des équipements coûteux.

La bride de maintien d'urgence du bassin doit toujours être utilisée en association avec les vis de réglage ou clous perforés du système, qui sont à usage unique. Dans l'exemple illustré sur la figure 3, la douille filetée 6 est vissée dans le trou de la partie finale d'un bras de tension et la vis de réglage 4 est insérée dans cette douille. L'extrémité de la vis de réglage 4 destinée à entrer en contact avec l'os du bassin est dotée d'une pointe 35. Un mauvais positionnement du clou dans l'os porotique, combiné avec une compression excessive de la vis sur l'os, pourraient entraîner une pénétration indésirable du clou dans l'os. En effet, quand les vis de réglage sont correctement placées dans les douilles, les bras supérieurs sont comprimés manuellement et la fixation finale est assurée en serrant les vis de réglage avec une clef avec ou sans cliquet. Dans cette dernière étape il existe donc un risque réel de perforation des parois osseuses du bassin pouvant entraîner une lésion d'un organe interne.

Le document US5300071 concerne un système de maintien des os du bassin dans lequel la bride est composée de deux parties circulaires qui coopèrent avec un mécanisme de serrage contenant des verrous qui permettent un mouvement rotationnel d'une partie circulaire par rapport à l'autre seulement dans un sens et pas dans l'autre. La compression du bassin n'étant pas limitée, ce dispositif risque de casser l'os du bassin. En outre les deux parties circulaires ne sont pas coulissantes l'une par rapport à l'autre, ce qui réduit les possibilités de réglage du dispositif.

Le document EP1488750 concerne une bride de maintien du bassin comprenant un système de crémaillère. La bride comprend au moins quatre parties : une pièce courbée et creuse, deux pièces coulissantes et une autre pièce courbée et creuse.

Le document US6162222 décrit une bride de fixation du bassin présentant trois points de fixation à chaque côté du bassin. Ce système réduit le risque de perforation de l'os, cependant il est compliqué et il requiert la fixation de six pointes. La bride peut être composée par deux pièces sensiblement orientées vers le haut télescopiques et une pièce moins trois pièces. En outre la pièce horizontale n'est pas composée par deux parties coulissantes l'une par rapport à l'autre pour le réglage.

Le document WO2006/126167 décrit un dispositif de maintien du bassin comprenant notamment deux demi-arceaux. La partie orientée vers le haut de chaque demi-arceau est composée de deux pièces qui coulissent l'une dans l'autre. Les parties horizontales des deux demi-arceaux sont en contact et elles sont bloquées par un mécanisme de serrage. Chaque demi-arceau a la même section sur la partie horizontale et sur celle orientée vers le haut. La section totale de la partie horizontale du dispositif est donc le double de la section des deux bras orientés vers le haut, ce qui engendre des angles des deux bras ayant la même ampleur mais une inclinaison opposée par rapport à la verticale, c'est-à-dire par rapport à la direction perpendiculaire au plan dans lequel le patient est couché sur le dos, qui pourraient causer un mouvement de rotation/torsion du dispositif. Ce système n'est donc pas suffisamment rigide.

Le document US20090264884 décrit un dispositif de fixation du bassin présentant deux fois deux arcs coulissant l'un dedans de l'autre.

Le document US5196012 concerne une bride de maintien du bassin dont le cadre, composé par un nombre de pièces significatives de deux, présente un système de réglage à crémaillère.

US5300071 décrit un dispositif de stabilisation du bassin avec deux arceaux pivotant autour une articulation centrale.

EP1488750 décrit un autre dispositif de maintien du bassin avec des sections diverses dans ses différentes parties.

US20080086122 décrit également un dispositif de maintien pour assister le réalignement des os fracturés.

Il apparait donc indispensable de mettre au point un dispositif de maintien du bassin simple à utiliser et qui permette un montage rapide.

Il existe donc un besoin pour un dispositif de maintien du bassin qui minimise le risque de perforation du bassin.

Il existe aussi un besoin pour un dispositif de maintien du bassin qui soit suffisamment rigide et qui évite des mouvements de rotation/torsion indésirables.

Il existe aussi un besoin pour un dispositif de maintien du bassin qui évite les opérations de démontage, vérification, nettoyage, stérilisation et lubrification après son usage et qui soit moins coûteux que les dispositifs existants.

### Bref résumé de l'invention

Un but de la présente invention est de proposer un dispositif de maintien du bassin exempt des limitations des dispositifs connus.

Selon l'invention, ces buts sont atteints notamment au moyen d'un dispositif de maintien du bassin selon la revendication 1.

Cette solution présente notamment l'avantage par rapport à l'art antérieur d'être simple à utiliser du fait qu'il est composé d'un minimum de pièces. En effet le dispositif comprend seulement deux pièces, qui dans une variante préférentielle sont identiques, et qui coulissent l'une par rapport à l'autre, au lieu des nombreux rails et bras de tension connus. Chaque pièce comprend une partie sensiblement horizontale et une partie orientée vers le haut. Dans ce contexte, l'adjectif « horizontal » se réfère au plan sur lequel le patient est couché sur le dos. Donc la partie sensiblement horizontale est sensiblement parallèle à ce plan. Dans ce contexte l'adverbe « sensiblement » indique que des valeurs par rapport à l'exacte verticalité ou horizontalité peuvent être tolérées. Dans ce contexte l'expression « vers le haut » indique une direction qui forme un angle par rapport au plan sur lequel le patient est couché sur le dos. Dans un exemple préférentiel la valeur de cet angle est comprise dans la plage 20° - 160°. Puisque la structure composée par les deux pièces peut pivoter autour des moyens pour serrer le dispositif au bassin, les parties orientées vers le haut ne sont pas nécessairement perpendiculaires à ce plan.

Avantageusement les deux pièces sont creuses, ce qui permet d'augmenter la rigidité en torsion et en flexion du dispositif.

La simplicité d'utilisation de ce dispositif implique aussi la rapidité de son montage : environ de 2 à 3 minutes sont nécessaires pour monter le dispositif objet de l'invention, ce qui permet un gain de temps qui, dans certains cas, peut être vital.

Dans une variante les deux pièces sont identiques puisqu'elles sont réalisées à partir d'un seul et même moule. La fabrication de ce dispositif étant plus simple, elle permet également un coût plus bas par rapport aux dispositifs existants.

Avantageusement, la section totale sensiblement horizontale, formée par les deux sections des parties sensiblement horizontales de chaque pièce qui coopèrent l'une par rapport à l'autre, est égale à chaque section des parties orientées vers le haut. Dans une variante préférentielle la partie sensiblement horizontale de chaque pièce a une section qui correspond sensiblement à la moitié de la section de la partie sensiblement orienté vers le haut. Dans une autre variante les deux pièces ne sont pas identiques, de façon à ce que par exemple la partie sensiblement horizontale d'une pièce a une section qui peut correspond à 1/3 de la section totale sensiblement horizontale et l'autre à 2/3. Toute autre répartition des sections entre les deux pièces peut être envisagée. Cependant cette solution est plus chère que la précédente puisque les deux pièces ne peuvent pas être réalisées par un seul et même moule.

Les parties sensiblement horizontales des deux pièces coopèrent l'une par rapport à l'autre dans le sens que, comme on le verra plus loin, elles coulissent l'une par rapport à l'autre au moins lors du réglage du dispositif. Il s'agit donc de parties coulissantes pour réglage.

Dans une variante préférentielle de l'invention, la section de la partie orientée vers le haut a une forme elliptique et donc celle de la partie sensiblement horizontale a une forme sensiblement semi-elliptique. Puisque la section totale sur la partie horizontale du dispositif est donnée par la somme des sections des parties sensiblement horizontales des deux pièces, cette caractéristique permet d'avoir un dispositif dans lequel la section est constante sur les parties orientées vers le haut et sur celles horizontales, ce qui donne au dispositif une meilleure rigidité que les solutions existantes et empêche des mouvements de rotation. Le dispositif est donc comparable à un seul arceau avec une section constante.

La rigidité du dispositif est donnée aussi par la présence, sur une surface de la partie sensiblement horizontale de chaque pièce, de dents, ou crans, et de nervures. La partie sensiblement horizontale d'une pièce coopère avec la partie sensiblement horizontale de l'autre pièce à travers ces dents et nervures : notamment les dents d'une pièce sont destinées à être engagées dans les nervures de l'autre pièce et vice-versa. Ce système à crémaillère permet aussi un premier réglage du dispositif en fonction de la corpulence du patient : en fonction de cette corpulence, les deux pièces peuvent être bougées et donc coulisser pour le réglage l'une par rapport à l'autre. Quand la largeur du dispositif ainsi réglé est adaptée à la corpulence du patient, les deux pièces sont rapprochées, de façon à ce que les dents d'une pièce soient en contact avec les nervures de l'autre pièce. Dans une variante préférentielle, le dispositif permettant le serrage des deux pièces est constitué d'une bague munie d'une vis ou de tout autre système de fixation ou de serrage rapide.

Le dispositif comprend deux moyens pour le serrage au bassin, chaque moyen coopérant avec la partie orientée vers le haut de chaque pièce. Avantageusement ces moyens permettent de minimiser le risque de perforation du bassin, parce qu'ils comprennent un système de limitation de la force de serrage ainsi qu'une rondelle grip munie d'une surface destinée à être en contact avec l'os du bassin ; cette rondelle est plus grande que celles connues dans l'état de la technique, ce qui permet de réduire la pression exercée sur l'os. Avantageusement cette surface est munie de plusieurs ergots pour l'ancrer à l'os du bassin. La rondelle est « grip » dans le sens qu'elle adhère à la surface de l'os avec laquelle elle est en contact.

La rondelle grip coopère avec une tige porte-rondelle au travers d'une rotule, ce qui permet au moyen de serrage du dispositif au bassin de s'adapter aux éventuelles concavités et/ou convexités de l'os.

Avantageusement le moyen de serrage du dispositif au bassin comprend un ressort destiné à limiter la force de serrage. Ce ressort est taré à la force maximale de serrage adapté à la qualité de l'os qui dans une variante est comprise dans la plage 20-40 kgf. Comme on le verra plus loin, deux variantes de ce moyen sont possibles : l'une plus facile à régler mais requérant une clef que le chirurgien doit utiliser pour le serrage, l'autre plus délicate à régler mais plus pratique pour le chirurgien qui peut serrer manuellement ce moyen.

Dans une variante préférentielle le dispositif est à usage unique : de cette façon, on évite toutes les opérations de démontage, vérification, nettoyage, stérilisation et lubrification après usage du dispositif. Dans une autre variante il peut être utilisé plusieurs fois.

Dans une variante préférentielle, les deux pièces du dispositif sont réalisées en un matériau radio-transparent, ce qui permet de réaliser un examen radiologique du patient sans retirer le dispositif. Dans une autre variante les deux pièces du dispositif sont réalisées en un matériau non ferreux, pour permettre une imagerie par résonance magnétique (IRM).

### Brève description des figures

Des exemples de mise en oeuvre de l'invention sont illustrés par les figures annexées dans lesquelles :
La figure 1 illustre une vue d'un dispositif du maintien du bassin connu ;
La figure 2 illustre les pièces qui composent un dispositif du maintien du bassin connu;
La figure 3 illustre une vis de réglage d'un dispositif du maintien du bassin connu ;
La figure 4 illustre une vue d'un mode de réalisation des deux pièces qui composent le dispositif objet de l'invention, ainsi que du moyen de serrage des deux pièces et des douilles filetées ;
La figure 5 illustre une vue simplifiée de la section de la partie sensiblement horizontale d'une pièce du dispositif objet de l'invention qui est creuse ;
La figure 6 illustre une vue simplifiée d'un mode de réalisation de la section de la partie orientée vers le haut d'une pièce du dispositif objet de l'invention qui est creuse ;
La figure 7 illustre une vue de dessus d'un mode de réalisation d'une surface de la section de la partie sensiblement horizontale d'une pièce du dispositif objet de l'invention ;
La figure 8 illustre une vue en coupe d'un mode de réalisation des parties sensiblement horizontales des deux pièces du dispositif objet de l'invention, ainsi que du moyen de serrage ;
La figure 9 illustre une vue simplifiée d'un premier mode de réalisation du moyen pour serrer le dispositif objet de l'invention au bassin ;
La figure 10 illustre un deuxième mode de réalisation du moyen pour serrer le dispositif objet de l'invention au bassin ;
Les figures 11 et 12 illustrent respectivement une vue latérale et une vue en coupe simplifiée du deuxième mode de réalisation du moyen pour serrer le dispositif objet de l'invention au bassin ;
La figure 13 illustre une extrémité du deuxième mode de réalisation du moyen pour serrer le dispositif objet de l'invention au bassin ;
La figure 14 illustre d'un mode de réalisation de la rondelle grip et de la tige porte-rondelle qui font partie du moyen pour serrer le dispositif objet de l'invention au bassin ;
La figure 15 illustre une vue en coupe simplifiée de la rondelle grip et de la tige porte-rondelle de la figure 14.

### Exemple(s) de mode de réalisation de l'invention

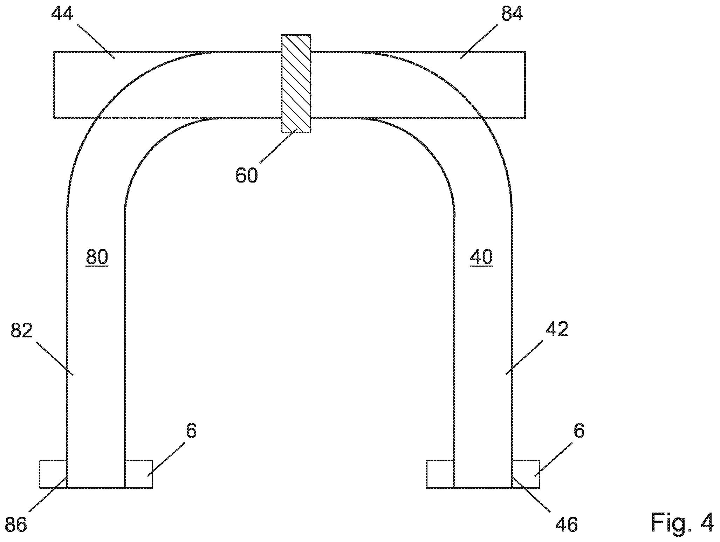

La figure 4 illustre une vue d'un mode de réalisation des deux pièces 40 et 80 du dispositif objet de l'invention, ainsi que le moyen de serrage 60 et les douilles filetées 6. Chaque pièce 40, 80 comprend une partie sensiblement horizontale 44 respectivement 84, c'est-à-dire sensiblement parallèle au plan du patient, et une partie orientée vers le haut 42 respectivement 82. Les directions horizontales et verticales se rapportent à un dispositif monté sur un patient couché sur une surface horizontale. Dans une variante préférentielle chaque partie est en forme de demi-arceau. Dans une autre variante préférentielle chaque partie est en forme de L. Les coins des parties finales des parties sensiblement horizontales 44 respectivement 84 sur la figure 4 sont représentés avec une forme en L, mais toute autre forme, y compris des formes arrondies, c'est-à-dire avec des coins arrondis, est envisageable.

Dans une variante préférentielle les deux pièces sont identiques puisqu'elles sont de préférence réalisées à partir d'un seul et même moule, ce qui permet de réduire les coûts de fabrication et donc d'avoir un dispositif moins cher par rapport aux solutions existantes et qui, donc, peut être à usage unique.

Dans une autre variante préférentielle les pièces sont réalisées en un matériau radio-transparent, par exemple un matériau composite à fibre longue de carbone et / ou de verre pour permettre de réaliser un examen radiologique du patient sans retirer le dispositif. L'usage des fibres de carbone ou de verre permet d'avoir un dispositif léger, rigide, solide et de réduire les coûts. Dans une autre variante les deux pièces du dispositif sont réalisées en un matériau non ferreux, pour permettre une imagerie par résonance magnétique (IRM).

Avantageusement les deux pièces sont creuses, ce qui permet un dispositif avec une rigidité plus élevée en torsion et en flexion que les solutions connues.

Avantageusement la section totale sensiblement horizontale du dispositif, formée par l'union des deux sections des parties sensiblement horizontales de chaque pièce, est égale à chaque section des parties orientées vers le haut. De cette façon le dispositif est comparable à un seul arceau avec une section constante, ce qui donne une rigidité supérieure à la structure et empêche des mouvements de rotation.

Dans une variante préférentielle la partie sensiblement horizontale 44, 84 de chaque pièce a une section qui correspond sensiblement à la moitié de la section de la partie orientée vers le haut 42, 82. L'adverbe « sensiblement » indique dans ce contexte que des tolérances, liées à la présence de dents et nervures sur les parties horizontales, peuvent être acceptées.

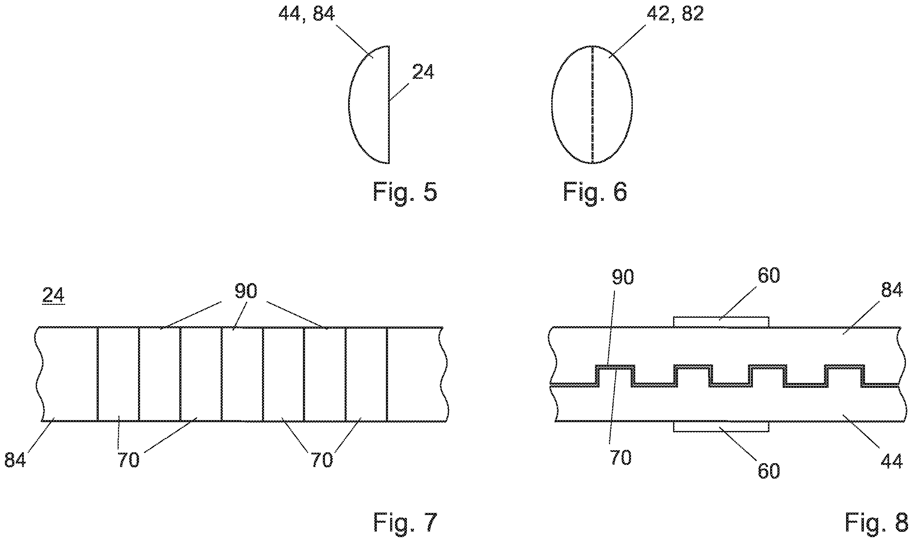

Une variante préférentielle de ces sections est illustrée sur les figures 5 et 6 : la section des parties orientées vers le haut 42, 82 dans cette variante est elliptique et donc celle des parties sensiblement horizontales 44, 84 est sensiblement semi-elliptique. Puisque les parties sensiblement horizontales de chaque pièce 40, 80 sont en contact entre elles à travers la surface plane 24 de leur section, cette caractéristique permet d'avoir la même section sur les parties orientées vers le haut et horizontales de la structure en forme d'arceau, ce qui donne une meilleure rigidité au dispositif que les solutions existantes. Pour un souci de clarté, les figures 5 et 6 illustrent une vue simplifiée de la section des parties sensiblement horizontales, qui sont creuses.

Dans une autre variante les deux pièces ne sont pas réalisées à partir d'une seul et même moule et toute répartition de la section totale sensiblement horizontale peut être envisagée.

Les deux parties sensiblement horizontales 44, 84 sont destinées à coopérer entre elles : comme discuté, les parties sensiblement horizontales de chaque pièce 40, 80 sont en contact entre elles à travers la surface plane 24 de leur section illustrée sur la figure 5. Avantageusement cette surface, qui par souci de clarté a été représentée avec une ligne droite sur la figure 5, est dotée de dents 70 et nervures 90, visibles sur la figure 7, qui représente une vue de dessus de cette surface 24. Quand les deux parties horizontales sont suffisamment éloignées, c'est-à-dire quand leur distance est supérieure à la hauteur des dents, elles peuvent coulisser l'une par rapport à l'autre pour un premier réglage du dispositif en fonction de la taille du patient. Les deux pièces donc sont coulissantes pour le réglage. Après avoir ajusté la distance entre les deux parties orientées vers le haut à la largeur du bassin, les deux parties horizontales sont rapprochées de façon à ce que les dents 70 d'une partie correspondent aux nervures 90 de l'autre partie. Il suffit qu'au moins trois dents soient bien placées pour que toutes les autres dents soient automatiquement bien engagées dans les nervures correspondantes. La présence des dents 70 et des nervures 90 améliore la rigidité du dispositif et évite que les deux pièces 40, 80 puissent glisser ou se tordre. Plus il y a de dents 70 et de nervures 90, meilleure est la possibilité de régler la largeur du dispositif.

Un moyen de serrage 60 serre les deux parties sensiblement horizontales 44, 84 entre elles. Dans la variante de la figure 4, ce moyen de serrage 60 comprend une bague avec une vis, ou tout autre système de fixation ou de serrage rapide qui peut être envisagé par l'homme du métier, non représentée. Par exemple les deux parties horizontales 44, 84 peuvent comprendre des trous et dans ce cas le moyen de serrage 60 peut comprendre une vis traversant deux trous alignés, chaque trou appartenant à une pièce, ainsi qu'une poignée pour le déclencher. Tout autre mécanisme pour bloquer les deux pièces l'une contre l'autre peut être envisagé.

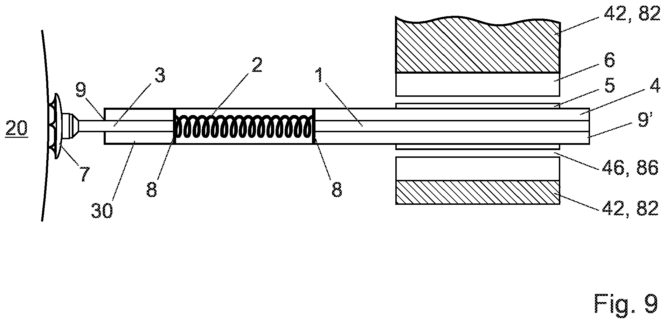

Chaque partie orientée vers le haut 42, 82 coopère avec un moyen 30 (visible sur les figures 9 à 12) pour serrer le dispositif au bassin. En effet l'extrémité de chaque partie orientée vers le haut 42, 82 est dotée d'un trou 46 respectivement 86 pour permettre l'insertion d'une douille filetée 6, dans lequel la vis de réglage 4 est vissée à l'aide du filetage 5.

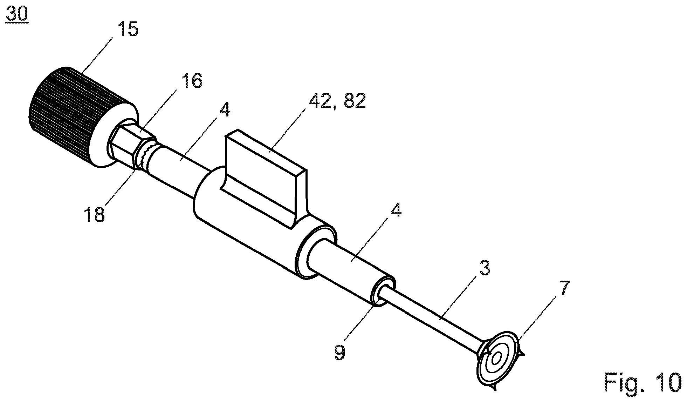

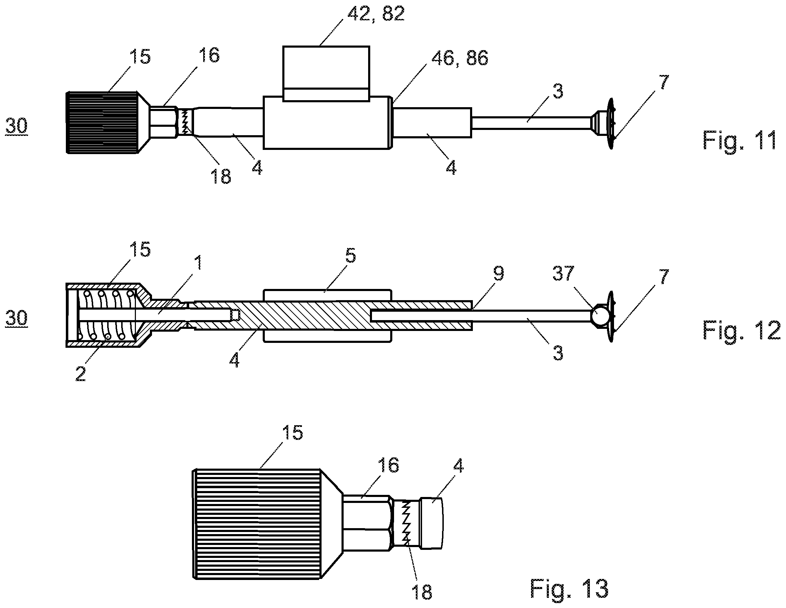

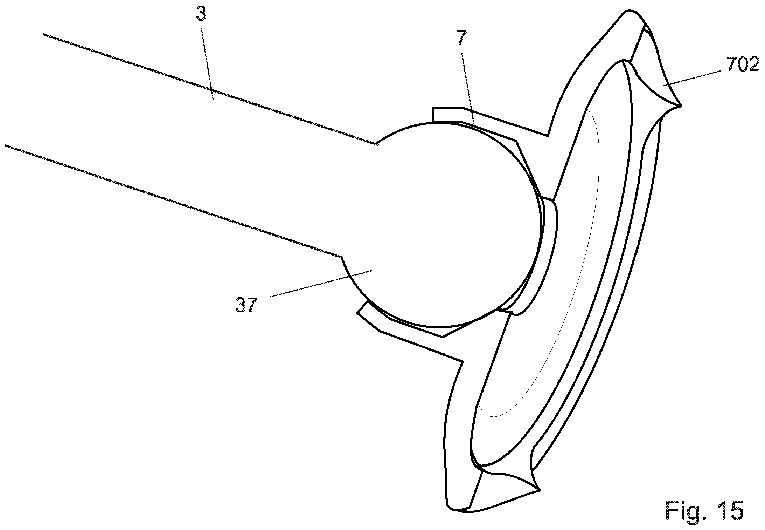

Il existe deux variantes de ce moyen 30 pour serrer le dispositif au bassin. La première variante est illustrée sur la figure 9, la deuxième sur les figures 10 à 13. Les deux variantes décrites ici comprennent
- un ressort 2 (figures 9 et 12) destiné à limiter la force de serrage du dispositif au bassin 20 et taré à une force de serrage comprise dans la plage 20-40 kgf
- une rondelle grip 7 munie d'ergots 702. Avantageusement la surface 700 de la rondelle est sensiblement plus grande que celle de la pointe 35 utilisée dans l'art antérieur de la figure 3. La rondelle grip est dotée d'un trou non représenté pour permettre, comme on le verra plus loin, l'insertion d'une broche de Kirschner.
- une tige porte-rondelle 3, munie d'une rotule 37 qui coopère avec la rondelle grip 7. Cette rotule est visible seulement sur les figures 12 et 15, qui illustrent une vue en coupe du moyen 30, puisqu'elle est contenue dans le moyen 30 ; elle est présente dans tous les moyens 30 représentés. La tige porte-rondelle est également dotée d'un trou non représenté pour permettre, comme on le verra plus loin, l'insertion d'une broche de Kirschner.

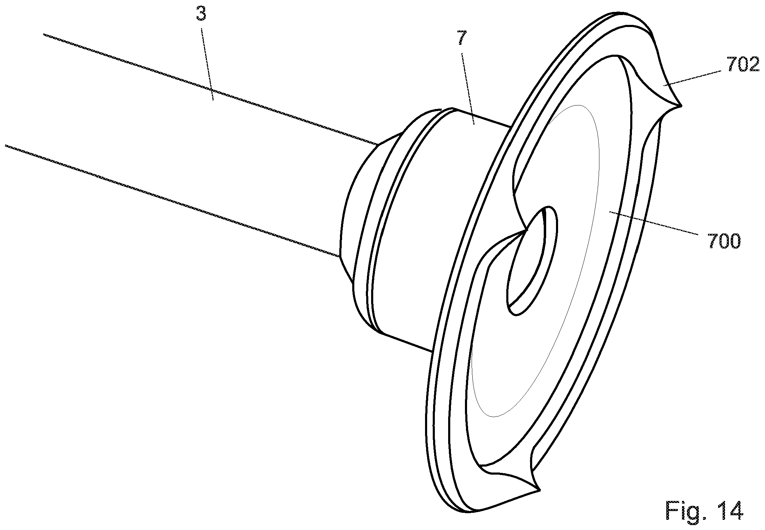

La tige porte-rondelle 3 ainsi que la rondelle grip 7 sont illustrées sur les figures 14 et 15. Les trous de la tige porte-rondelle 3 ainsi que de la rondelle grip 7, qui permettent le passage d'une broche de Kirschner, pour un souci de clarté sur ces figures n'ont pas été représentés. La présence de la surface 700 en contact avec l'os 20 au lieu de la pointe 35 permet de réduire la pression exercée sur l'os et donc de diminuer le risque de percer le bassin. Avantageusement la surface 700 est munie de plusieurs ergots 702 pour l'ancrer à l'os du bassin 20, visibles sur les figures 14 et 15.

La rondelle grip 7 coopère avec une tige porte-rondelle 3 à travers une rotule 37, ce qui permet un mouvement relatif entre la tige 3 et la rondelle 7 pour adapter le moyen 30 aux éventuelles concavités et/ou convexités de l'os 20.

Une première version du moyen 30 est illustrée sur la figure 9. Il comprend :
- le ressort 2
- la rondelle grip 7
- la tige porte-rondelle 3
- deux moyens 8 destinés à entrer en contact avec les extrémités du ressort 2 : dans la variante illustrée, ces moyens sont constitués par des surfaces, mais d'autres solutions, comprenant des billes, peuvent être envisagées; ces moyens également peuvent être dotés d'un trou non représenté pour permettre l'insertion d'une broche de Kirschner
- une tige coulissante 1 liée à une extrémité du ressort 2, qui également peut être dotée d'un trou non représenté pour permettre l'insertion d'une broche de Kirschner
- une douille filetée 6 qui est insérée dans le trou 46 ou 86 d'une pièce est qui est destiné à recevoir la vis 4
- la vis de réglage filetée 4 avec un trou six pans, comprenant le ressort 2, les deux moyens 8 et la tige coulissante 1. A noter que la référence 5 sur la figure 9 indique le filetage de la vis 4 qui coopère avec la douille filetée 6.

Pour pouvoir monter le dispositif 10, après le réglage manuel des deux pièces 40, 80 en fonction de la taille du patient tel que discuté ci-dessus, la douille filetée 6 doit être insérée dans chaque trou 46, 86 des deux pièces 40 respectivement 80. La vis de réglage filetée 4, comprenant le ressort 2, les surfaces 8 et la tige coulissante 1, est donc vissée dans la douille filetée 6. La tige porte-rondelle 3 liée à la rondelle grip 7 est donc insérée dans le trou 9 de la vis 4 jusqu'à entrer en contact avec une surface 8.

Dans une variante préférentielle la rondelle grip 7 et la tige porte-rondelle 3 présentent un trou central non représenté pour pouvoir recevoir une broche de Kirschner qui assure le positionnement correct du moyen 30 dans le corps du patient. Dans une autre variante la broche de Kirschner est glissée dans toute la longueur du moyen 30, à travers un trou central non représenté.

Après l'incision du patient sur les deux côtés au niveau de l'os du bassin, une tige de Kirschner est fixée de chaque côté provisoirement dans l'os pour repérer la position du moyen 30. Une partie des deux moyens 30 est donc insérée de chaque côté dans le corps du patient en utilisant la broche de Kirschner comme guide jusqu'à ce que la rondelle grip 7 entre en contact avec l'os 20. Le moyen de serrage 30 est en fixé par une clef ou un cliquet par le chirurgien pour serrer la vis de réglage filetée 4. En la vissant, le ressort 2 se comprime en repoussant la tige coulissante 1 vers l'extérieur, c'est-à-dire vers le coté opposé au corps du patient. Le ressort 2 est taré de façon à ce que lorsqu'il arrive à sa force maximale, l'extrémité de la tige coulissante 1 affleure l'ouverture 9' de la vis 4. En dépassant la force de serrage maximale du ressort 2, la tige coulissante 1 sort de la vis 4 de façon à ce que la clef ne puisse plus pénétrer dans le trou à six pans de la vis 4 et donc le chirurgien n'a plus aucune possibilité de serrer cette vis 4.

Cette variante évite donc d'exercer une force supérieure à la force maximale à laquelle le ressort est taré. Par contre elle requiert que le chirurgien utilise une clef pour le serrage.

Une deuxième variante du moyen 30 est représentée sur les figures 10 à 12. Dans ce cas elle comprend :
- un tambour 15
- le ressort 2, qui est contenu dans le tambour 15 et donc, à différence de la première version de la figure 9, il n'est plus contenu dans la vis 4
- une douille 16 connectée au tambour 15 et qui coopère avec la vis de réglage filetée 4 à travers une denture débrayable à la force de serrage 18
- la rondelle grip 7
- la tige porte-rondelle 3
- une tige 1 liée au tambour 15. A noter que dans ce cas cette tige ne coulisse pas
- une douille filetée 6 qui est insérée dans le trou 46 ou 86 d'une pièce et qui est destiné à recevoir la vis 4
- la vis de réglage filetée 4 qui coopère avec la douille filetée 6. Dans ce cas la vis n'est pas creuse, mais elle contient des cavités destinées à recevoir la tige 1 et la tige porte-rondelle 3. Elle coopère avec la douille 16 à travers la denture 18.

Le réglage manuel des deux pièces 40, 80 en fonction de la taille du patient, l'insertion de la douille filetée 6 dans chaque trou 46, 86 des deux pièces 40 respectivement 80, le vissage de la vis de réglage filetée 4 dans la douille filetée 6 et l'insertion de la tige porte-rondelle 3 liée à la rondelle grip 7 dans le trou 9 de la vis 4 sont les mêmes que dans la première variante. Les considérations sur la broche de Kirschner restent valides.

Comme dans la première variante, après l'incision du patient sur les deux côtés au niveau de l'os du bassin, une partie des deux moyens 30 est insérée dans le corps du patient jusqu'à ce que la rondelle grip 7 entre en contact avec l'os 20. Le moyen de serrage 60 est donc serré, par exemple en vissant une vis. Au lieu d'utiliser une clef ou un cliquet pour serrer la vis de réglage filetée 4, le chirurgien dans ce cas agit manuellement sur le tambour 15 en le tournant. Le ressort 2 est donc comprimé et la douille 16 est mise en rotation par rapport à la vis 4 à travers la denture 18. Quand le ressort 2 atteint la force maximale à laquelle il peut être taré, la denture 18 se bloque et le tambour 15 tourne à vide.

A la différence de la première version, l'application d'une force supérieure à celle autorisée est difficile mais pas impossible. Cependant, cette variante est plus pratique pour le chirurgien qui n'a pas besoin d'un outil supplémentaire comme une clef pour le serrage.

### Numéros de référence employés sur les figures

- 1: Tige
- 2: Ressort
- 3: Tige porte-rondelle
- 4: Vis de réglage filetée
- 5: Filetage de la vis de réglage
- 6: Douille filetée
- 7: Rondelle grip
- 8: Moyen destiné à entrer en contact avec les extrémités du ressort
- 9: Trou de la vis 4 destiné à recevoir la tige porte-rondelle 3
- 9': Ouverture de la vis 4 (côté opposé au corps du patient)
- 10: Dispositif de maintien du bassin
- 15: Tambour
- 16: Douille
- 18: Denture débrayable au couple
- 20: Bassin
- 24: Surface de la section de la partie sensiblement horizontale d'une pièce
- 30: Moyen pour serrer le dispositif de maintien du bassin au bassin
- 35: Pointe du moyen pour serrer le dispositif de maintien du bassin au bassin connue dans l'état de la technique
- 37: Rotule
- 40: Première pièce du dispositif
- 42: Partie orientée vers le haut de la première pièce du dispositif
- 44: Partie sensiblement horizontale de la première pièce du dispositif
- 46: Trou de la première pièce du dispositif
- 60: Moyen de serrage
- 64: Ultérieure pièce horizontale d'un dispositif connu
- 70: Dents
- 80: Deuxième pièce du dispositif
- 82: Partie orientée vers le haut de la deuxième pièce du dispositif
- 84: Partie sensiblement horizontale de la deuxième pièce du dispositif
- 86: Trou de la deuxième pièce du dispositif
- 90: Nervures
- 700: Surface de la rondelle grip
- 702: Ergot de la rondelle grip

## Revendications

1. Dispositif de maintien du bassin (10) comprenant
- deux pièces (40, 80), chaque pièce (40 ; 80) comprenant une première partie (44; 84) sensiblement parallèle, en position d'utilisation, à un plan horizontal défini par le patient couché et une deuxième partie (42 ; 82) orientée vers le haut, en position d'utilisation par rapport au dit plan horizontal, les premières parties (44; 84) des deux pièces étant coulissantes l'une par rapport à l'autre pour le réglage dudit dispositif
- un moyen de serrage (60) desdites deux pièces qui coopère avec la première partie (44 ; 84) de chaque pièce
- deux moyens (30) pour serrer ledit dispositif audit bassin (10), chaque moyen (30) coopérant avec la deuxième partie (42 ; 82) de chaque pièce (40 ; 80),
**caractérisé en ce que**
- la section totale formée par les deux sections des premières parties coulissantes l'une par rapport à l'autre de chaque pièce est égale à chaque section des deuxièmes parties.

2. Le dispositif de maintien du bassin selon la revendication 1, dans lequel les deux pièces (40, 80) sont identiques.

3. Le dispositif de maintien du bassin selon la revendication 2, dans lequel ladite section de ladite deuxième partie (42 ; 82) est elliptique.

4. Le dispositif de maintien du bassin selon l'une des revendications 1 à 3, dans lequel lesdits moyens (30) pour serrer ledit dispositif audit bassin comprennent une tige porte-rondelle (3) et une rondelle grip (7) comprenant une surface (700) munie d'ergots (702) destinée à entrer en contact avec ledit bassin (20).

5. Le dispositif de maintien du bassin selon la revendication 4, dans lequel ladite rondelle grip (7) coopère avec ladite tige porte-rondelle (3) au travers d'une rotule (37).

6. Le dispositif de maintien du bassin selon l'une des revendications 1 à 5, dans lequel lesdits moyens (30) pour serrer ledit dispositif audit bassin (20) comprennent un ressort (2) destiné à limiter la force de serrage dudit dispositif audit bassin (20).

7. Le dispositif de maintien du bassin selon l'une des revendications 1 à 6, dans lequel ladite première partie (44; 84) comprend des dents (70) et des nervures (90).

8. Le dispositif de maintien du bassin selon l'une des revendications 1 à 7, dans lequel lesdites deux pièces (40 ; 80) sont réalisées en matériau radio-transparent.

9. Le dispositif de maintien du bassin selon l'une des revendications 1 à 8, dans lequel lesdits moyens (30) pour serrer ledit dispositif audit bassin (20) comprennent une vis de réglage filetée (4), un filetage (5) et une douille filetée (6) insérée dans un trou (46, 86) d'une des deux dites pièces (40; 80).

10. Le dispositif de maintien du bassin selon la revendication 9, ladite vis de réglage filetée (4) comprenant un trou six pans, ledit ressort (2), des moyens (8) destinés à entrer en contact avec les extrémités dudit ressort (2) et une tige (1) liée à une extrémité dudit ressort (2).

11. Le dispositif de maintien du bassin selon la revendication 10, lesdits moyens (8) destinés à entrer en contact avec les extrémités dudit ressort (2) comprenant des surfaces et/ou des billes.

12. Le dispositif de maintien du bassin selon la revendication 9, dans lequel lesdits moyens (30) pour serrer ledit dispositif audit bassin (20) comprennent un tambour (15), une douille (16) et une denture débrayable à la force de serrage (18) qui coopère avec ladite tige filetée (4).

13. Le dispositif de maintien du bassin selon la revendication 12, dans lequel ledit ressort (2) est contenu dans ledit tambour (15).

14. Le dispositif de maintien du bassin selon l'une des revendications 1 à 13, dans lequel ledit moyen de serrage (60) comprend une bague et un système de fixation.

15. Le dispositif de maintien du bassin selon l'une des revendications 1 à 14, dans lequel lesdites pièces (40, 80) sont en forme de demi-arceaux.

16. Le dispositif de maintien du bassin selon l'une des revendications 1 à 15, dans lequel lesdites pièces (40, 80) sont creuses.

## Patentansprüche

1. Beckenhaltevorrichtung (10) mit:
- zwei Elementen (40, 80), wobei jedes Element (40; 80) einen ersten Teil (44; 84) umfasst, welcher im Wesentlichen in der Betriebsstellung parallel zur vom liegenden Patienten definierten waagrechten Ebene ist, und einen zweiten Teil (42; 82), welcher in der Betriebsstellung nach oben in Bezug auf die besagte waagrechte Ebene orientiert ist, wobei die ersten Teile (44; 84) der zwei Elemente in Bezug auf einander für die Einstellung der besagten Vorrichtung schiebbar sind,
- einem Mittel (60) zum Anziehen der besagten zwei Elemente, welches mit dem ersten Teil (44; 84) jedes Elements zusammenarbeitet,
- zwei Mitteln (30) zum Anziehen der besagten Vorrichtung auf das besagte Becken (10), wobei jedes Mittel (30) mit dem zweiten Teil (42; 82) jedes Elements (40; 80) zusammenarbeitet,
**dadurch gekennzeichnet, dass**
- der gesamte, durch die zwei Querschnitte der ersten in Bezug auf einander schiebbaren Teile jedes Elements gebildete Querschnitt gleich jedem Querschnitt der zweiten Teile ist.

2. Beckenhaltevorrichtung gemäss Anspruch 1, worin die zwei Elemente (40, 80) identisch sind.

3. Beckenhaltevorrichtung gemäss Anspruch 2, worin der besagte Querschnitt des besagten zweiten Teils (42; 82) elliptisch ist.

4. Beckenhaltevorrichtung gemäss einem der Ansprüche 1 bis 3, worin die besagten Mittel (30) zum Anziehen der besagten Vorrichtung auf das besagte Becken eine scheibentragende Stange (3) und eine Griffscheibe (7) umfassen mit einer mit Nocken (702) versehenen Fläche (700), welche dazu bestimmt ist, mit dem besagten Becken (20) in Kontakt zu kommen.

5. Beckenhaltevorrichtung gemäss Anspruch 4, worin die besagte Griffscheibe (7) mit der besagten scheibentragenden Stange (3) über einen Kugelkopf (37) zusammenarbeitet.

6. Beckenhaltevorrichtung gemäss einem der Ansprüche 1 bis 5, worin die besagten Mittel (30) zum Anziehen der besagten Vorrichtung auf das besagte Becken (20) eine Feder (2) umfassen, welche für die Einschränkung der Anziehkraft der besagten Vorrichtung auf das besagte Becken (20) bestimmt ist.

7. Beckenhaltevorrichtung gemäss einem der Ansprüche 1 bis 6, worin der besagte erste Teil (44; 84) Zähne (70) und Rippen (90) umfasst.

8. Beckenhaltevorrichtung gemäss einem der Ansprüche 1 bis 7, worin die besagten zwei Elemente (40; 80) aus strahlendurchlässigem Material bestehen.

9. Beckenhaltevorrichtung gemäss einem der Ansprüche 1 bis 8, worin die besagten Mittel (30) zum Anziehen der besagten Vorrichtung auf das besagte Becken (20) eine gewindete Einstellschraube (4), ein Gewinde (5) und eine Gewindehülse (6), die in ein Loch (46, 86) von einem der zwei besagten Elemente (40; 80) eingefügt ist, umfassen.

10. Beckenhaltevorrichtung gemäss Anspruch 9, worin die besagte gewindete Einstellschraube (4) einen Sechskantenloch, die besagte Feder (2), Mittel (8), welche dazu bestimmt ist, mit den Enden der besagten Feder (2) in Kontakt zu kommen, und eine mit einem Ende der besagten Feder (2) verbundene Stange (1) umfasst.

11. Beckenhaltevorrichtung gemäss Anspruch 10, worin die besagten Mittel (8) welche dazu bestimmt sind, mit den Enden besagten Feder (2) in Kontakt zu kommen, Flächen und/oder Kugeln umfassen.

12. Beckenhaltevorrichtung gemäss Anspruch 9, worin die besagten Mittel (30) zum Anziehen der besagten Vorrichtung auf das besagte Becken (20) eine Trommel (15), eine Hülse (16) und eine Zahnung (18), welche in lösbarer Wirkungsverbindung zur Anziehkraft steht und mit der besagten gewindete Einstellschraube (4) zusammenarbeitet, umfassen.

13. Beckenhaltevorrichtung gemäss Anspruch 12, worin die besagte Feder (2) innerhalb der besagten Trommel (15) enthalten ist.

14. Beckenhaltevorrichtung gemäss einem der Ansprüche 1 bis 13, worin die besagten Anziehmittel (60) einen Ring und ein Befestigungssystem umfassen.

15. Beckenhaltevorrichtung gemäss einem der Ansprüche 1 bis 14, worin die besagten Elemente (40, 80) die Form von Halbbögen aufweisen.

16. Beckenhaltevorrichtung gemäss einem der Ansprüche 1 bis 15, worin die besagten Elemente (40, 80) hohl sind.

## Claims

1. Pelvis holding device (10) comprising:
- two elements (40, 80), wherein each element (40; 80) comprises a first portion (44; 84) essentially parallel, when in its position of use, to a horizontal plane defined by the recumbent patient and a second portion (42; 82) oriented upwards, in its position of use, relative to said horizontal plane, wherein the first portions (44; 84) of the two elements are capable of sliding one relative to the other for adjusting said device,
- a tightening means (60) of said two elements that cooperates with the first portion (44; 84) of each element,
- two means (30) for tightening said device onto said pelvis (10), wherein each means (30) cooperates with the second portion (42; 82) of each element (40; 80),
**characterized in that**
- the total section formed by the two sections of the first portions sliding one relative to the other of each element is equal to each section of the second portions.

2. Pelvis holding device according to claim 1, wherein the two elements (40, 80) are identical.

3. Pelvis holding device according to claim 2, wherein said section of said second portion (42; 82) is elliptical.

4. Pelvis holding device according to one of the claims 1 to 3, wherein said means (30) for tightening said device onto said pelvis comprise a disc-bearing rod (3) and a grip disc (7) comprising a surface (700) provided with spikes (702) designed to come into contact with said pelvis (20).

5. Pelvis holding device according to claim 4, wherein said grip disc (7) cooperates with said disc-bearing rod (3) via a ball joint (37).

6. Pelvis holding device according to one of the claims 1 to 5, wherein said means (30) for tightening said device to said pelvis (20) comprise a spring (2) designed to limit the tightening force of said device to said pelvis (20).

7. Pelvis holding device according to one of the claims 1 to 6, wherein said first portion (44; 84) comprises teeth (70) and grooves (90).

8. Pelvis holding device according to one of the claims 1 to 7, wherein said two elements (40; 80) are made of radiolucent material.

9. Pelvis holding device according to one of the claims 1 to 8, wherein said means (30) for tightening said device to said pelvis (20) comprise a threaded adjustment screw (4), a threading (5) and a threaded sleeve (6) inserted in a hole (46, 86) of one of the two said elements (40; 80).

10. Pelvis holding device according to claim 9, wherein said threaded adjustment screw (4) comprises a hexagonal hole, said spring (2), means (8) designed to come into contact with the extremities of said spring (2) and a rod (1) connected to one extremity of said spring (2).

11. Pelvis holding device according to claim 10, wherein said means (8) designed to come into contact with the extremities of said spring (2) comprise surfaces and/or balls.

12. Pelvis holding device according to claim 9, wherein said means (30) for tightening said device to said pelvis (20) comprise a drum (15), a bushing (16) and a toothing (18) that can be disengaged to the tightening force which cooperates with said threaded rod (4).

13. Pelvis holding device according to claim 12, wherein said spring (2) is contained within said drum (15).

14. Pelvis holding device according to one of the claims 1 to 13, wherein said tightening means (60) comprises a ring and a fastening system.

15. Pelvis holding device according to one of the claims 1 to 14, wherein said elements (40, 80) are in the shape of half-arcs.

16. Pelvis holding device according to one of the claims 1 to 15, wherein said elements (40, 80) are hollow.
